# EUROPEAN PATENT APPLICATION

(11) **EP 2 287 324 A2**
(43) Date of publication of application: **23.02.2011**
(21) Application number: 10178179.7
(22) Date of filing: 01.04.2004
(51) Int. Cl.: C12P 1/00, C12P 1/04, C12P 19/62, C12R 1/01, C12R 1/04, C12R 1/465

(54) **Fermentation processes with low concentrations of carbon- and nitrogen-containing nutrients**

(30) Priority: 04.04.2003 EP 03100896
(62) Divisional of application: 04725049.3
(71) Applicant: DSM IP Assets B.V., 6411 TE Heerlen (NL)
(72) Inventor: Hollander, De, Johannes Abraham, 2343 NE, Oegstgeest (NL); Eswilder, Freddy Ronald, 2645 HE, Delfgauw (NL); Zanden, Van Der, Paulus Petrus Maria, 2641 MS, Pijnacker (NL)
(74) Representative: Biermann, Jan

(57) **Abstract**

The present invention describes a fermentation process for the production of a desired compound (such as natamycin) comprising cultivating a filamentous bacterial strain in a liquid fermentation medium, wherein the carbon containing nutrients and nitrogen containing nutrients are maintained at low concentrations in the fermentation medium. The process of the invention reduces the viscosity of the culture medium and therefore increases the yield of the desired compound.

## Description

### Field of the invention

The present invention relates to the field of fermentative production of desired compounds, such as secondary metabolites, proteins or peptides.

### Background of the invention

The *actinomycetes,* a family of filamentous bacteria, are of great importance for the fermentation industry. Many members of this family are known to produce secondary metabolites or extracellular enzymes and several of these products of bacterial metabolism have an industrial application. For obtaining these products, the bacteria are generally cultivated in liquid media (submerged cultures), leading to excretion of the products into the liquid, from which they can be isolated. Formation of product can take place during the initial fast growth of the organism and/or during a second period in which the culture is maintained in a slow-growing or non-growing state. The amount of product which is formed per unit of time during such a process (the productivity) is generally a function of a number of factors: the intrinsic metabolic activity of the organism; the physiological conditions prevailing in the culture (e.g. pH, temperature and medium composition); and the amount of organisms which are present in the equipment used for the process. Generally, during optimisation of a fermentation process, it is preferred to obtain a concentration of bacteria that is as high as possible because, assuming that the intrinsic productivity per unit of organism is a constant, the highest titer of product will be obtained. However, one particular characteristic of the bacteria which belong to the family of *actinomycetes,* makes it difficult to achieve this goal. *Actinomycetes,* when grown in submerged culture, have a filamentous morphology, which generally leads to highly viscous culture fluids. A high viscosity of the culture limits the rate of oxygen transfer to the culture. Virtually all processes utilising *actinomycetes* depend on the presence and consumption of oxygen and therefore a limitation in oxygen transfer will impose a limitation on the overall process productivity. The viscosity of a culture fluid is determined by a number of factors such as the composition of the medium, the presence and nature of products excreted by the microorganisms, and (most important) the morphology of the microorganism. If one could influence the morphological characteristics of the microorganisms in a positive way (i.e. to decrease the specific viscosity), the process could be operated at a higher production rate or a higher concentration of bacteria could be achieved. Both changes in the process would result in a higher productivity.

### Summary of the invention

The present invention provides a fermentation process for the production of a desired compound comprising culturing a filamentous bacterial strain in a liquid fermentation medium, wherein the carbon containing nutrients and nitrogen containing nutrients are maintained at low concentrations in the fermentation medium.

Preferably, a feed comprising carbon and nitrogen containing nutrients is supplied to the medium and the nutirients in the feed are in such a ratio that low concentrations of both carbon and nitrogen containing nutrients are maintained in the culture.

The filamentous bacteria are preferably of the family *Actinomyces,* more preferably of the genus *Streptomyces.*

### Detailed description of the invention

Surprisingly, it has been found that certain medium compositions lead to a reduced culture viscosity of a fermentation process comprising a filamentous bacterial strain, without affecting the production of the desired compound. An important factor appears to be the ratio of nitrogen containing nutrients (N) to carbon containing nutrients (C) in the medium. A high N/C ratio (relative excess of nitrogen compounds) leads to viscous cultures, whereas a low N/C ratio results in relatively low viscosity of the culture fluid. When the amount of nitrogen in the medium is restricted too much, this leads to very poor growth of the organism and low amounts of product are formed. However, at an intermediate N/C ratio, growth of the organism is good and product formation is normal, while the morphology of the organism is apparently changed in such a way that the viscosity of the culture fluid is significantly reduced. The consequence of this finding is, that by carefully controlling the medium, or more specifically by controlling the ratio of carbon and nitrogen containing nutrients in the medium, a process comprising a filamentous bacterial strain can be improved significantly.

Bacterial strains of the family *Actinomycetes* are known to produce desired compounds, which have commercial applications, such as secondary metabolites, proteins and peptides. Examples thereof are natamycin, nistatine, glucose isomerase and clavulanic acid.

For example, the *actinomycetes* strains *Streptomyces natalensis* and *Streptomyces silvosporens* produce the antifungal compound natamycin, which has several applications as an antifungal compound. Fermentation processes comprising such filamentous bacteria are generally characterised by two phases. Usually the process starts with a phase where growth of the microorganism occurs until conditions for growth become unfavourable, for instance because one of the growth supporting nutrients becomes depleted from the medium. The initial (batch) phase may be followed by a phase where the microorganisms are maintained in a viable state. Often most of the product of interest is formed in this second phase. In this second phase, more nutrients may be supplied to the culture, either discontinuously as a single or repeated charge of fresh nutrients, or continuously by feeding one or more nutrients containing fluids in to the fermentation vessel. This mode of fermentation is called fed-batch fermentation. Preferably, a fermentation process may be further prolonged by removing part of the fermentation mash, for instance when the fermentation vessel becomes completely filled as a result of feeding with nutrient containing fluids. This process form is called extended fermentation or repeated (fed-)batch fermentation.

The initial (batch) phase will end when one of the nutrients is depleted. This phase may be followed by measuring the oxygen uptake which will decrease towards the end of the initial phase. In general, the initial phase will take 6 to 48 hours. The second phase starts when feeding of the nutrients is started. Feeding of nutrients allows the continuation of the fermentation process for a longer period than is possible in simple batch fermentation process.

In general, for each production process, the optimal ratio of carbon and nitrogen containing nutrients can be determined by the skilled person, depending on the elementary composition of the organism and the product(s), the effect of the N/C ratio on the physiology of the organism and, more specifically, the product forming capacity of the organism. It has been found that neither carbon excess nor nitrogen excess will lead to the desired result. In the optimal situation, both the available carbon and nitrogen will be almost depleted from the medium at the end of the batch process and/or during the process of prolonged fed-batch type fermentation. The concentration of the nitrogen containing nutrient in the medium during the second phase is preferably less than 0.5 g/l, more preferably less than 0.25 g/l and most preferably less than 0.1 g/l (expressed as gram of nitrogen per litre). The concentration of the carbon containing nutrient is preferably less than 5 g/l, more preferably less than 2.5 g/l and most preferably less than 1 g/l (expressed as gram of carbon per litre). The feed can be supplied as one feed containing all the nutrients or preferably as more than one subfeeds each comprising either a nitrogen containing nutrient, a carbon containing nutrient or a combination of nitrogen and carbon containing nutrients.

The feed is also controlled in such a way that the amount of oxygen is between 20 and 70% of air saturation, preferably between 30 and 60% of air saturation.

Oxygen, typically in the form of air, is generally introduced at or near the bottom of the fermentor. One of more nozzles are installed for the introduction of air or another oxygen containing gas such as (purified) oxygen.

Optionally, a stirrer is present in the reactor to stimulate the oxygen uptake. Moreover, the stirrer prevents concentration gradients of the feed or subfeed developing in the fermentor.

### Legend to the Figures

Figure 1: Viscosity development of a nitrogen excess-culture (•) and a nitrogen-carbon double-limited culture (◆).
Figure 2: Agitation power required to control the dissolved oxygen concentration at a 30% air saturation. Both cultures, nitrogen excess (•) and nitrogen-carbon double-limited (◆), were operated under otherwise similar process conditions.
Figure 3: Viscosity development of a nitrogen excess culture (•) and a nitrogen-carbon double-limited culture (◆).
Figure 4: Product accumulation in a nitrogen excess culture (•) and a nitrogen-carbon double-limited culture (◆).
Figure 5: Full scale fermentation of *Streptomyces natalensis* to produce natamycin. The initial process (•) used a limiting feed of soybean oil, while the NH3 concentration was kept at a non-limiting level. In the improved process (◆) the NH3 concentration was kept at a low value by continuous feeding of a NH3 solution in proportion to the oil feeding rate.

The reduced culture viscosity allowed faster feeding of oil. The increase in product formation was approximately proportional to the increase in oil feeding rate.

### EXAMPLES

### Example 1

*Steptomyces natalensis* strain ATCC27448 was cultivated in 2000ml conical shake containing 500 mL growth medium of the following composition:

| | g/L |
|---|---|
| Glucose.1H₂O | 30 |
| Casein hydrolysate | 15 |
| Yeast Extract (dried) | 10 |
| De-foamer Basildon | 0.4 |

The pH was adjusted to 7.0 by adding NaOH/H₂SO₄, and the medium was sterilized by autoclavation (20 minutes at 120°C). The content of a full-grown shake flask was used to inoculate a fermentation vessel containing 6L medium of the following composition:

| | g/L |
|---|---|
| Soybean flower | 25 |
| Soybean oil | 8 |
| Corn Steep (dried) | 1 |
| KH₂PO₄ | 0.45 |
| Trace element solution | 17 |
| De-foamer Basildon | 0.4 |

The composition of the trace element solution was as follows:

| | g/L |
|---|---|
| Citric acid.1H₂O | 175 |
| FeSO₄.7H₂O | 5.5 |
| MgSO₄.7H₂O | 100 |
| H₃BO₃ | 0.06 |
| CuSO₄.5H₂O | 0.13 |
| ZnSO₄. 7H₂O | 1.3 |
| CoSO₄.7H₂O | 0.14 |

The temperature and pH of the medium were controlled at 25°C and 7.0 respectively. Dissolved oxygen concentration was kept above 30% of air saturation, by increasing airflow and/or stirrer speed when necessary. After preliminary growth in batch culture for approximately 24 hours the culture entered the second phase of fermentation. During the second phase, the growth and product formation were continued by feeding pure soybean oil. A second feeding line was installed to feed ammonia. The average feeding rate of the soybean oil was 3 g/h. Ammonia was supplied in proportion to the soybean oil feeding rate. A series of fermentations were carried out, in which different ammonia feeding rates were applied while keeping the soybean oil feeding rate constant. For this strain, the carbon source and the nitrogen source were totally consumed when the ratio of NH3 to oil was in the range of 30-40 mg NH3/g oil. This condition of C-N double limitation resulted in cultures with the lowest specific viscosities. Nitrogen excess (NH3/oil ratio >40 mg/g) resulted in a considerable increased viscosity of the culture. Carbon excess (NH3/oil ratio <30 mg/g) had a similar effect. In addition, the accumulation of oil had a negative effect on the culture viability. The range of ratios of nitrogen containing nutrients versus carbon containing nutrients is dependent on the strain and the nature of the nitrogen and carbon sources. For every new process, the optimal range can therefore be determined by the present procedure.

Two experiments were carried out according to the process described above. One experiment was aimed to reach a condition of nitrogen excess (i.e. the culture is then purely limited by the soybean oil feeding rate). In another experiment the rate of ammonia feeding relative to soybean oil feeding was reduced, in order to arrive at a condition where the concentration of both nutrients (soybean oil and ammonia) in the fermenter vessel is very low. For the test organism *(Strepromyces natalensis)* in the chosen conditions, the ratio of ammonia feeding rate relative to the oil-feeding rate should be around 35 mg NH₃ per g oil. The ammonia surplus experiment was carried out at a ratio of 45 mg NH₃ per g oil.

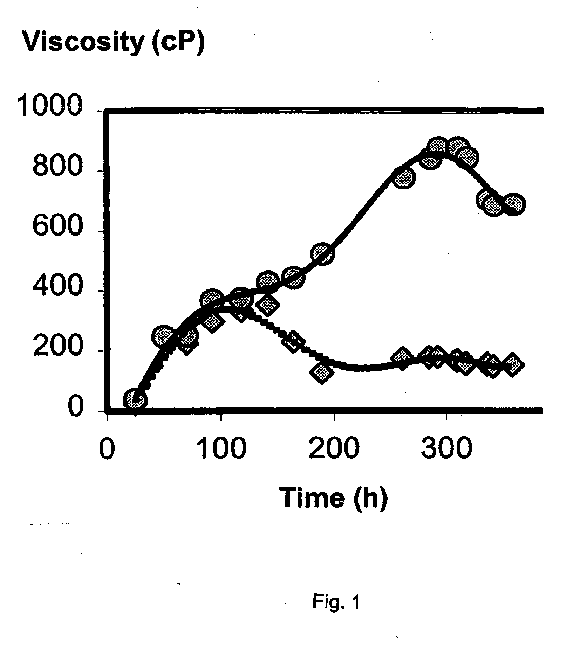

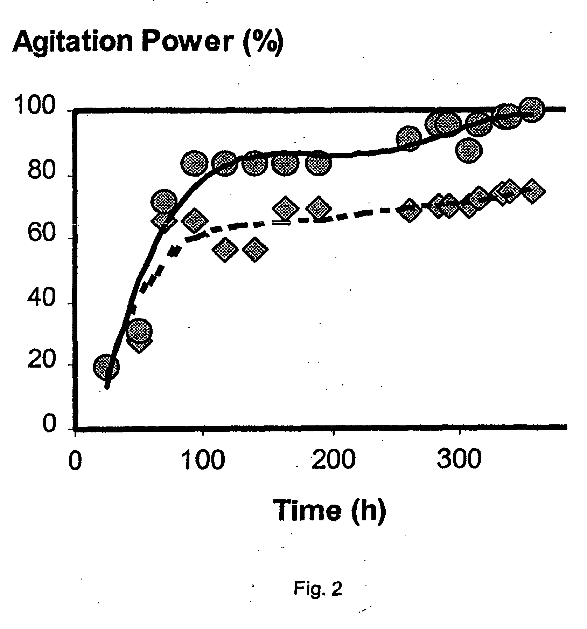

The effect of the carbon-nitrogen double limitation is clearly demonstrated in Figure 1. Under nitrogen excess conditions the viscosity reaches the usual high values. Under conditions of simultaneous carbon and nitrogen limitation, the viscosity drops to a much lower value, causing better aeration conditions. For a good production it is preferred that the dissolved oxygen concentration is maintained at a level of above 30% of air saturation. Figure 2 illustrates that for maintaining this dissolved oxygen concentration much less agitation power (energy) is needed when the culture is under a condition of nitrogen-carbon double limitation.

### Example 2

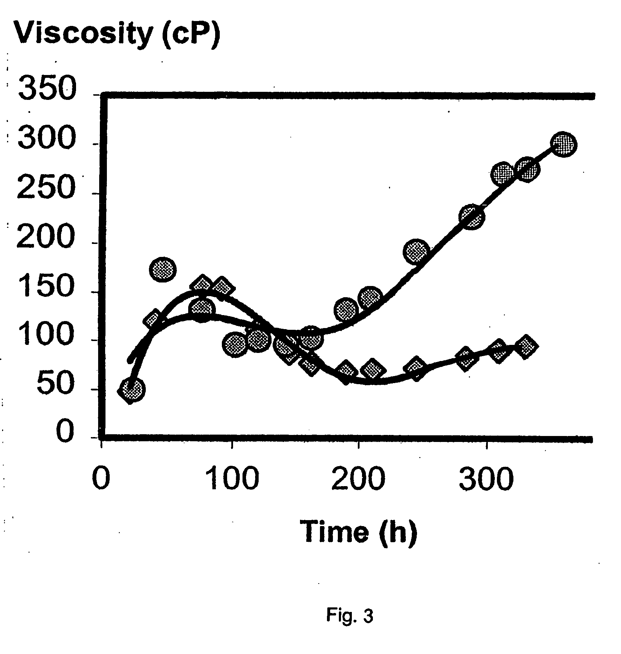

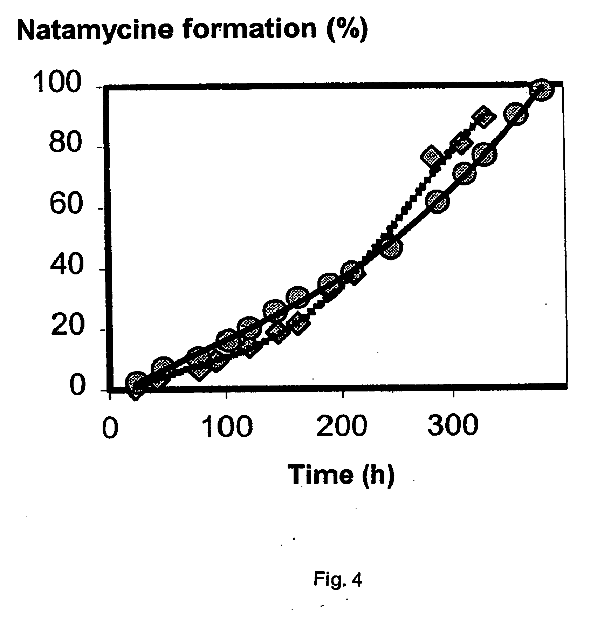

Another fermentation experiment was carried out using the same procedure as described in Example 1 using a strain of *Streptomyces natalensis.* This strain is a producer of the anti-fungal compound natamycin. In this experiment two fermentations were run. One experiment was under carbon limitation and nitrogen excess (NH₃ level was kept at 150-200 mg/L during the oil feeding phase). The second experiment was run under nitrogen-carbon double limitation during the oil feeding phase, employing a NH₃/oil ratio of 32 mg/g. Some results are shown in Figure 3 and 4. It is obvious that a very significant difference in viscosity was observed between the two modes of fermentation. A low viscosity is very beneficial for efficient process operation. However, a low viscosity coupled with a poor product formation potency would be negative. In this experiment, the product formation was not affected at all by the conditions leading to low viscosity (Figure 3). The rate of product formation in the nitrogen-carbon double limitation experiment is faster in the second part of the fermentation despite a slightly slower start.

### Example 3

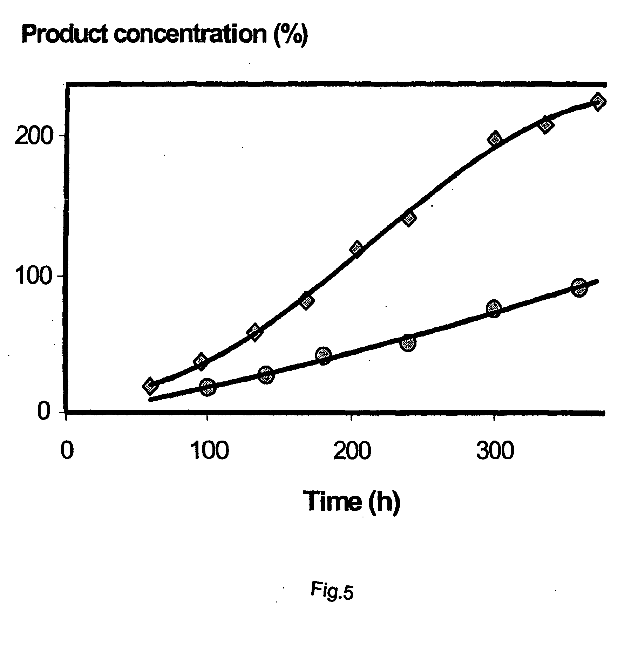

The information obtained in the experiments described in Examples 1 and 2 was used to improve the actual production process of natamycin on an industrial scale (100m³ scale). The reduced viscosity allows intensification of the process by faster feeding of the main nutrient soybean oil. The feeding rate of NH3 was proportional to the feeding of oil, as described in the Examples 1 and 2, resulting in carbon-nitrogen double limitation during the feeding phase (which started at about 24 hours after inoculation of the fermentation vessel). The process conditions and medium composition were similar to the small scale experiments described in Examples 1 and 2. Starting with a small increase, the oil feeding rate was increased step-wise from run to run, until a process intensity was reached which could just be maintained on minimal dissolved oxygen tension. Figure 5 illustrates, the improvement in product output resulting from the higher oil feeding rate was quite substantial.

## Claims

1. A fermentation process for the production of a desired compound comprising cultivating a filamentous bacterial strain in a liquid fermentation medium, wherein the carbon containing nutrients and nitrogen containing nutrients are maintained at low concentrations in the fermentation medium.

2. A fermentation process according to claim 1, wherein the concentration of the nitrogen containing nutrient in the medium is less than 0.5 g/l (expressed as gram of nitrogen per litre).

3. A fermentation process according to claim 1 or 2, wherein the concentration of the carbon containing nutrient in the medium is less than 5 g/l (expressed as gram of carbon per litre).

4. A fermentation process according to any one of claims 1 to 3, wherein a feed comprising carbon containing nutrients and nitrogen containing nutrients is supplied to the medium and wherein the nutrients in the feed are in such a ratio that low concentrations of both carbon and nitrogen containing nutrients are maintained in the culture.

5. A fermentation process of any one of claims 1 to 4, wherein the feed is supplied to the medium via more than one subfeed and wherein each subfeed comprises nitrogen containing nutrients, carbon containing nutrients or a combination of nitrogen and carbon containing nutrients.

6. A fermentation process according to any one of claims 1 to 4, wherein the amount of oxygen in the medium is between 20 and 70% of air saturation.

7. A fermentation process according to claim 6, wherein the amount of oxygen in the medium is between 30 and 60% of air saturation.

8. A process according to any one of claims 1 to 7, wherein the bacteria are of the family of *Actinomycetes.*

9. A process according to any claim 8, wherein the bacteria are of the genus *Streptomyces.*

10. A process according to claim 9, wherein the bacteria are *Streptomyces natalensis* or *Streptomyces gilvosporeus* and wherein the desired compound is natamycin.

11. A fermentation process according to anyone of claim 1 to 10, wherein the carbon containing nutrient is for more than 50% soybean oil (calculated as gram of carbon) and the nitrogen containing nutrient is for more than 50% ammonia (calculated as gram of nitrogen).
